# FASCICULE DE BREVET EUROPEEN

(11) **EP 0 755 706 B1**
(45) Date de publication et mention de la délivrance du brevet: **27.12.2006**
(21) Numéro de dépôt: 96401533.3
(22) Date de dépôt: 11.07.1996
(51) Int. Cl.: B01D 3/00, B01J 8/02, B01J 8/04

(54) **Procédé et dispositif de distillation réactive avec distribution particulière des phases liquide et vapeur**
Verfahren und Vorrichtung zur reaktiven Destillation mit besonderer Verteilung von den flüssigen und gasförmigen Fasen
Process and apparatus for reactive distillation with particular distribution of the liquid and vapour phases

(30) Priorité: 24.07.1995 FR 9509060; 27.12.1995 FR 9515532
(43) Date de publication de la demande: 29.01.1997
(73) Titulaire: Institut Français du Pétrole, 92502 Rueil-Malmaison (FR)
(72) Inventeur: Marion, Marie-Claire, 69100 Villeurbanne (FR); Viltard, Jean-Charles, 26000 Valence (FR); Travers, Philippe, 92500 Rueil Malmaison (FR); Harter, Isabelle, 69008 Lyon (FR); Forestiere, Alain, 69390 Vernaison (FR)

(56) Documents cités:
- EP-A- 0 231 841
- EP-A- 0 470 655
- EP-A- 0 571 163
- CH-A- 366 519
- DE-A- 1 801 538

## Description

L'invention concerne un procédé de distillation réactive (appelée aussi distillation catalytique), qui permet d'effectuer à la fois au moins une réaction chimique en présence de catalyseur et la séparation par distillation du mélange réactionnel obtenu. L'invention concerne aussi un dispositif de distillation réactive.

Le procédé et le dispositif de l'invention peuvent s'appliquer à diverses réactions équilibrées, en phase liquide, pour lesquelles on peut isoler le produit de réaction par distillation dans les conditions de température et de pression auxquelles on réalise la réaction, et particulièrement à la préparation d'éthers alkyliques tertiaires tels que le méthyltertiobutyléther (MTBE), le méthyltertioamyléther (TAME), l'éthyltertiobutyléther (ETBE), ou encore l'éthyltertioamyléther (TAEE), par réaction catalytique sur l'oléfine appropriée (isobutène ou isopentène) de l'alcool approprié (méthanol ou éthanol, suivant le cas). En ce qui concerne plus particulièrement les réactions d'éthérification par distillation réactive, divers procédés ont déjà été proposés dans l'art antérieur.

Le brevet US-A-4.847.430 décrit un procédé d'étherification dont une des caractéristiques est la disposition dans le(s) lit(s) catalytique(s) de passages ou cheminées pour la vapeur de distillation ascendante, permettant d'éviter les inconvénients que procurerait le passage de ladite vapeur au sein du lit catalytique.

Le brevet US-A-5.130.102 décrit une colonne de distillation réactive comprenant une zone dans laquelle sont effectuées concurremment une réaction catalytique et la séparation par distillation des produits de ladite réaction, cette zone comportant au moins un "plateau" de réaction supportant un catalyseur particulaire et au moins un plateau de distillation, plus particulièrement du type plateau à calottes, ledit plateau de réaction étant "couplé" avec ledit plateau de distillation au moyen d'une masse liquide continue immergeant en sa zone inférieure ledit catalyseur et en sa zone supérieure ledit plateau de distillation. Le plateau de réaction comporte une cheminée (en fait c'est la cheminée de la calotte qui a été étendue pour tenir compte de la présence de la masse catalytique et de sa hauteur), destinée à amener la vapeur montant de la zone située au-dessous de l'ensemble "couplé" vers un distributeur de vapeur situé au-dessous du plateau de distillation. La hauteur de la masse liquide entre le plateau de réaction et le plateau de distillation doit être suffisante pour éviter l'entraînement des particules du catalyseur. La phase liquide s'écoule de la partie supérieure de l'ensemble couplé par débordement par dessus un déversoir puis par l'intermédiaire d'une descente soit vers un plateau de distillation classique, soit vers un autre ensemble "couplé" situé au-dessous de l'ensemble "couplé" considéré. De même, le plateau de réaction d'un ensemble "couplé" est alimenté en phase liquide par une descente provenant, par débordement par-dessus un déversoir, soit d'un plateau de distillation classique soit d'un autre ensemble "couplé", situé au-dessus de l'ensemble "couplé" considéré.

Le brevet US-A-5.368.691 décrit un procédé mettant en jeu une configuration telle que les zones de réaction, alternant avec des zones de distillation, sont nettement séparées de ces dernières, c'est-à-dire sans masse liquide continue entre une zone de réaction et la zone de distillation voisine. Ladite demande décrit un procédé de distillation réactive caractérisé en ce que :
o on introduit les réactifs, généralement séparément ou en mélange, purs ou dilués, en au moins un niveau d'une colonne de distillation réactive qui renferme :
   (a) au moins une zone réactionnelle catalytique renfermant un lit du catalyseur approprié ; et
   (b) au moins une zone de distillation constituée par au moins un plateau de distillation, chaque zone de distillation étant séparée de la (ou des) zone(s) catalytique(s) consécutive(s);
o on maintient des conditions de distillation dans ladite colonne de façon à avoir une phase liquide et une phase vapeur dans ladite colonne ;
o on fait circuler une partie, de préférence la totalité, de la phase liquide du bas vers le haut à travers le catalyseur dans chaque zone catalytique;
o on fait circuler une partie, de préférence la totalité, de la phase vapeur de la distillation du bas vers le haut à travers chaque zone de distillation, de telle manière que ladite phase vapeur soit en contact avec la phase liquide dans les zones de distillation;
o on recueille une partie, de préférence la totalité, du produit recherché à une extrêmité de ladite colonne de distillation réactive; et
o on recueille une partie, de préférence la totalité, du diluant éventuel des réactifs et de tout excès de réactif(s) à l'autre extrémité de ladite colonne.

De manière préférée, on fait circuler la totalité de la phase vapeur de la distillation de manière qu'elle ne soit en contact avec la phase liquide que dans les zones de distillation et non dans les zones catalytiques.

Le procédé et le dispositif selon l'invention, explicités ci-après, constituent un perfectionnement du procédé et du dispositif décrits dans le brevet US-A-5.368.691.

Le procédé et le dispositif de l'invention peuvent s'appliquer à diverses réactions équilibrées, en phase liquide, pour lesquelles on peut isoler le produit de réaction par distillation dans les conditions de température et de pression auxquelles on réalise la réaction. Ils peuvent s'appliquer aux réactions d'alkylation d'hydrocarbures aromatiques, généralement le benzène, par réaction catalytique avec l'oléfine appropriée, par exemple l'éthylène ou le propylène, pour former l'alkylbenzène correspondant, par exemple l'éthylbenzène ou le cumène. Mais ils peuvent aussi s'appliquer par exemple aux réactions d'isomérisation de paraffines, aux réactions d'isomérisation d'oléfines, ou à la production de butène par hydro-isomérisation de butène-1 en butène-2. Ils peuvent s'appliquer aussi à la réaction d'hydrogénation du benzène et des oléfines légères (ayant 6 atomes de carbone au plus dans leur molécule) dans le réformat léger, ou à la réaction d'hydrogénation des dioléfines et/ou des composés acétyléniques dans des fractions légères des coupes pétrolières ou pétrochimiques. De façon préférée, ils s'appliquent aux réactions d'éthérification entre une isooléfine (par exemple l'isobutène et l'isopentène) et un monoalcool aliphatique (tel que le méthanol ou l'éthanol), particulièrement à la préparation d'éthers alkyliques tertiaires tels que le méthyltertiobutyléther (MTBE), le méthyltertioamyléther (TAME), l'éthyltertiobutyléther (ETBE), ou encore l'éthyltertioamyléther (TAEE), par réaction catalytique sur l'oléfine appropriée (isobutène ou isopentène) de l'alcool approprié (méthanol ou éthanol, suivant le cas).

Le procédé de distillation réactive de l'invention est tel que :
o - on introduit les réactifs éventuellement dilués par un diluant, l'un des réactifs pouvant être excédentaire, en au moins un niveau d'une zone de distillation réactive qui renferme :
   a) au moins une zone réactionnelle catalytique' renfermant au moins un lit catalytique comportant le catalyseur,
   b) au moins une zone de distillation située au-dessous de la zone catalytique précédente, ou zone de distillation inférieure,
   c) au moins une autre zone de distillation située au-dessus de la zone catalytique précédente, ou zone de distillation supérieure,
o on maintient des conditions de distillation de façon à avoir une phase liquide et une phase vapeur dans la zone de distillation réactive,
o on fait circuler la majeure partie, de préférence la quasi-totalité, du liquide du bas vers le haut à travers le catalyseur dans la zone catalytique,
o on fait circuler la majeure partie de la vapeur du bas vers le haut dans la zone catalytique, de telle manière que ladite vapeur ne soit pas en contact avec le catalyseur,
o on recueille une partie, de préférence la majeure partie, du produit de réaction recherché,
o on recueille une partie, de préférence la majeure partie, d'un diluant éventuel et des réactifs,
o on récupère la majeure partie du liquide après son passage dans le lit catalytique par au moins un moyen de déversement du liquide, de façon à ce que le liquide se déverse dans la zone de distillation inférieure,
o et on collecte la majeure partie du liquide issu de la zone de distillation supérieure,
ledit procédé étant caractérisé en ce que :
o on collecte la majeure partie du liquide issu de la zone de distillation supérieure par au moins un moyen collecteur de façon à ce que le liquide arrive en zone centrale de la zone catalytique, au-dessous de chaque lit catalytique de la zone catalytique, sans avoir de contact avec le catalyseur,
o on fait circuler sensiblement radialement la majeure partie du liquide au-dessous dudit lit catalytique par au moins un moyen de circulation sensiblement radiale de façon à l'introduire dans au moins une zone de répartition du liquide.

Dans le procédé selon l'invention, la zone de distillation réactive consiste en une zone réactionnelle catalytique, une zone de distillation inférieure qui est la zone d'épuisement et une zone de distillation supérieure qui est la zone de rectification.

Le moyen collecteur peut être tel que le liquide arrive pratiquement exempt de vapeur au-dessous dudit lit catalytique, c'est-à-dire que ledit moyen prend toute forme adéquate pour réaliser une alimentation de la zone de répartition par au moins un moyen de circulation sensiblement radiale du liquide correcte, évitant en particulier tout gaz entraîné dans ledit liquide. Ainsi, le liquide se répartit sans turbulences excessives dans le moyen de circulation sensiblement radiale puis dans la zone de répartition.

La zone de répartition du liquide, alimentée par au moins un moyen de circulation sensiblement radiale, permet la régulation et l'homogénéisation du liquide avant son entrée dans chaque lit catalytique de la zone catalytique. Ainsi un des avantages de la présente invention est d'améliorer la circulation du liquide dans chaque lit catalytique de la zone catalytique. Par suite, le procédé est tel que le liquide s'étale sur toute la section de la zone de distillation réactive qui lui est accessible avant d'entrer dans le lit catalytique, dans au moins une zone de répartition du liquide.

De manière préférée, le liquide issu de la zone de distillation supérieure qui est collecté circule en majeure partie en zone centrale de la zone catalytique.

De manière préférée, le liquide qui se déverse dans la zone de distillation inférieure passe par au moins un moyen de déversement en majeure partie en zone périphérique de la zone catalytique.

Lorsque la réaction mise en jeu comprend la présence d'au moins un réactif gazeux, le procédé selon l'invention peut comporter au moins une introduction dudit réactif gazeux, de préférence l'hydrogène, pour chaque lit catalytique de la zone catalytique.

Dans un premier mode de réalisation préféré du procédé selon l'invention, le moyen collecteur est situé en zone centrale de la zone catalytique, le liquide issu de la zone de distillation supérieure qui est collecté circule en majeure partie en zone centrale de la zone catalytique, le liquide qui se déverse dans la zone de distillation inférieure passe en majeure partie en zone périphérique de la zone catalytique, et la vapeur issue de la zone de distillation inférieure et qui circule dans la zone catalytique circule principalement en zone centrale de la zone catalytique. Dans ce cas, de préférence, le moyen de circulation sensiblement radiale de chaque lit catalytique comporte au moins un bras de distribution, de préférence de 2 à 10 bras de distribution, de manière encore plus préférée de 2 à 8 bras de distribution.

Dans le premier mode de réalisation préféré du procédé selon l'invention, la vapeur issue de la zone de distillation inférieure, qui circule en zone centrale de la zone catalytique, passe en quasi totalité en zone centrale de la zone catalytique. Dans ledit mode, le moyen de déversement du liquide est situé en zone périphérique de la zone catalytique et de préférence ledit moyen est tel qu'il ne permet pratiquement pas de passage de phase vapeur dans le sens ascendant.

De plus, de manière préférée, dans ledit mode de réalisation, la vapeur issue de la zone de distillation inférieure et qui circule en zone centrale de la zone catalytique, n'est pratiquement pas en contact avec du liquide qui circule en zone centrale de ladite zone catalytique.

Dans un second mode de réalisation préféré du procédé selon l'invention, le moyen collecteur est situé en zone centrale de la zone catalytique, le liquide issu de la zone de distillation supérieure qui est collecté circule en majeure partie en zone centrale de la zone catalytique, le liquide qui se déverse dans la zone de distillation inférieure passe en majeure partie en zone périphérique de la zone catalytique, et la vapeur issue de la zone de distillation inférieure et qui circule dans la zone catalytique circule en zone périphérique de la zone catalytique. Alors, un premier mode de réalisation qui est donc un troisième mode de réalisation préféré du procédé selon l'invention est tel que la vapeur issue de la zone de distillation inférieure et qui circule en zone périphérique de la zone catalytique est au moins en partie, de préférence en majeure partie, en contact avec le liquide circulant dans le moyen de déversement. Et un second mode de réalisation qui est donc un quatrième mode de réalisation préféré du procédé selon l'invention est tel que la vapeur issue de la zone de distillation inférieure et qui circule en zone périphérique de la zone catalytique n'a sensiblement pas de contact avec le liquide circulant dans le moyen de déversement.

Quel que soit le mode de réalisation du procédé selon l'invention, le moyen de circulation sensiblement radiale peut comporter au moins un bras de distribution, de préférence de 2 à 10 bras de distribution, de manière encore plus préférée de 2 à 8 bras de distribution.

Bien entendu, la vapeur produite lors de la réaction catalytique dans chaque lit catalytique de la zone catalytique rejoint au-dessus dudit lit catalytique la vapeur issue de la zone de distillation inférieure qui traverse ledit lit catalytique sous contact avec le catalyseur, quel que soit le mode de réalisation.

Tout autre mode de réalisation du procédé selon l'invention, combinant les différents modes de réalisation préférés du procédé selon l'invention, est aussi envisageable. Par exemple, il est possible de combiner les troisième et premier modes de réalisation préférés décrits précédemment, c'est-à-dire qu'une partie de la vapeur issue de la zone de distillation inférieure circule en zone périphérique de la zone catalytique et que l'autre partie de ladite vapeur circule en zone centrale de la zone catalytique.

La zone de distillation réactive selon l'invention est au moins une colonne catalytique. Dans les cas connus de l'homme du métier où la mise en oeuvre d'une seule colonne pose des problèmes, on préfère généralement scinder ladite colonne de façon à utiliser finalement au moins deux colonnes qui, mises bout à bout, réalisent ladite colonne, c'est-à-dire que les zones de rectification, de distillation catalytique et d'épuisement se répartissent sur les colonnes. En pratique, la zone de rectification ou la zone d'épuisement, et de préférence la zone d'épuisement, peut généralement se trouver dans au moins une colonne différente de la colonne comprenant la zone catalytique.

La zone supérieure ou zone de rectification de la zone de distillation réactive comporte généralement soit au moins un plateau distributeur éventuellement situé au-dessus d'une zone comportant au moins un garnissage assurant la distillation, soit au moins un plateau de distillation, qui peut être conventionnel, ainsi que toute combinaison de garnissage(s), de plateau(x) distributeur(s) et de plateau(x) de distillation assurant la distillation selon toute technique connue de l'homme du métier.

De même, la zone d'épuisement de la zone de distillation réactive comporte généralement soit au moins un plateau distributeur éventuellement situé au-dessus d'une zone comportant au moins un garnissage assurant la distillation, soit au moins un plateau de distillation, qui peut être conventionnel, ainsi que toute combinaison de garnissage(s), de plateau(x) distributeur(s) et de plateau(x) de distillation assurant la distillation selon toute technique connue de l'homme du métier.

Le corps de garnissage éventuellement utilisé dans le procédé de la présente invention est choisi en fonction de l'efficacité nécessaire à la fonction distillation. Le corps de garnissage peut être choisi parmi les corps de garnissage bien connus de l'homme du métier, tels que par exemple des solides sous forme d'anneaux, d'extrudés polylobés ou de selles. A titre d'exemple non limitatif de corps de garnissage, on peut citer les anneaux de Raschig, les anneaux de Pall, les anneaux d'Intos, les selles de Berl, les selles Novalox et les selles Intalox. Mais le corps de garnissage peut aussi être choisi parmi les garnissages structurés, par exemple de type FLEXIPAC (marque déposée) commercialisés par la société Koch, ou SULZER CHEMTECH ou SULZER (marques déposées) commercialisés par la société Sulzer.

Le plateau distributeur éventuellement utilisé dans le procédé de la présente invention est un simple plateau permettant le passage ascendant de la vapeur et la collecte puis le déversement de liquide, tel que tout plateau distributeur connu de l'homme du métier, et tout particulièrement si la zone de distillation à laquelle il appartient comprend en majeure partie des garnissages.

Dans le cas où l'on utilise, selon le procédé de la présente invention, un plateau de distillation, celui-ci peut-être choisi parmi les plateaux de distillation connus de l'homme de l'art, notamment les plateaux perforés, les plateaux à calottes, les plateaux à clapets, et il comporte :
o au moins une table de travail discontinue, c'est-à-dire munie de discontinuités pour le passage de la phase vapeur, chaque table de travail étant destinée au brassage et au mélange des courants liquide et vapeur,
o au moins une descente (ou goulotte), de préférence située au centre ou à la périphérie de chaque plateau de distillation, pour le conditionnement du liquide et le contrôle de la régularité de son écoulement (descente à travers laquelle va s'écouler le liquide se trouvant auparavant sur la table de travail dudit plateau), et
o au moins un déversoir (ou petite margelle) bordant chaque descente, destiné au maintien d'un certain niveau de liquide sur la table de travail dudit plateau et donc au contrôle de la régularité de l'évacuation du liquide de ladite table de travail.

Selon la présente invention, il est par exemple envisageable que toute zone de distillation située entre deux lits catalytiques comprenne un nombre impair de plateaux de distillation, de préférence à double passe (alternance de plateau(x) à moyen de déversement en partie central et de plateau(x) à moyen(s) de déversement en partie périphérique(s), ainsi qu'il est bien connu de l'homme du métier).

Le catalyseur à installer dans chaque lit catalytique de zone catalytique est généralement constitué de particules catalytiques. Il peut être conditionné sous toute forme adéquate, notamment sous forme de particules de forme sensiblement cylindrique ou de forme sensiblement sphérique, les dimensions desdites particules de catalyseur étant généralement comprises entre 0,1 et 20 mm. La nature dudit catalyseur dépend de la réaction envisagée, ainsi qu'il est bien connu de l'homme du métier. Dans le cas de la synthèse de méthyltertiobutyléther par exemple, on utilise en général un catalyseur du type résine sulfonique, par exemple une résine polystyrène-divinylbenzène sulfonée, dont la granulométrie est généralement comprise entre 0,1 et 1,2 mm.

Dans chaque lit catalytique de la zone catalytique, le catalyseur peut être enfermé dans au moins une enveloppe perméable au liquide mais imperméable aux particules catalytiques (c'est-à-dire ne laissant pas passer lesdites particules solides de catalyseur), enveloppe constituée par exemple par une toile en tissu, en matériau synthétique (par exemple en polypropylène) ou en tissu métallique.

Le catalyseur peut également être disposé en vrac, c'est-à-dire librement, à l'intérieur de chaque lit catalytique de la zone catalytique. Dans ce cas, pour maintenir le catalyseur en place et pour éviter qu'il soit entraîné par le courant de liquide qui le traverse, on prévoit généralement que tout lit catalytique compris dans la zone catalytique repose sur tout dispositif permettant le passage du liquide mais imperméable aux particules catalytiques, telles que par exemple une grille de type Johnson ou des busettes de type Johnson réparties régulièrement sur une surface imperméable aux flux gazeux, liquides ou solides. Avantageusement, ledit dispositif est légèrement surélevé par rapport à l'arrivée du (des) moyen(s) de circulation sensiblement radiale, sur le fond de la zone catalytique, de manière à créer une zone de répartition située en partie au-dessous du lit catalytique de la zone catalytique.

Il est possible aussi de prévoir la présence d'une grille supérieure sur tout lit catalytique compris dans la zone catalytique, imperméable au passage des particules catalytiques, perméable au liquide et au gaz, de façon à confiner lesdites particules dans ledit lit. Mais il peut être aussi envisagé, et c'est un mode de réalisation préféré de l'invention, de mettre en place tout dispositif permettant le passage du liquide mais imperméable aux particules catalytiques, au niveau du moyen de déversement par lequel s'écoule le liquide après son passage en zone catalytique, telle qu'une grille de type Johnson. De même, il est aussi possible de prévoir la présence d'une grille supérieure en haut du moyen de circulation de la vapeur, imperméable aux particules catalytiques et perméable à la vapeur ascendante qui traverse le lit catalytique pratiquement sans avoir de contact avec les dites particules, de facon à prévenir un entraînement éventuel de catalyseur lors d'un fonctionnement anormal de la colonne.

Lorsque le catalyseur est disposé en vrac, on peut prévoir de l'utiliser sous forme de lit fixe, de lit expansé ou de lit fluidisé. Que le catalyseur soit disposé en vrac ou enfermé dans au moins une enveloppe, le taux de vide que l'on peut lui conférer est compris généralement entre 30 et 70%.

Lors du fonctionnement de la zone de distillation réactive, dans le procédé selon l'invention, en raison de l'entraînement possible par le liquide de fragments de particules catalytiques constituant le catalyseur (ou fines), il peut être avantageux de prévoir de collecter le liquide s'écoulant par exemple du lit catalytique inférieur de la zone catalytique située le plus bas dans la zone catalytique, de soutirer ce liquide, de le filtrer dans au moins un dispositif de filtrage (situé généralement à l'extérieur de la zone de distillation réactive), et de le réintroduire dans la zone catalytique entre la position d'où il a été soutiré et la zone de distillation inférieure. Le lit catalytique inférieur est l'unique lit de ladite zone catalytique si celle-ci ne comporte qu'un seul lit catalytique, ou bien le lit catalytique situé le plus bas de la zone catalytique si celle-ci comporte au moins deux lits catalytiques. Ce filtrage permet d'éliminer tous les fragments éventuels de catalyseur entraînés par la phase liquide et, en particulier, les empêche de venir en fond de la zone de distillation réactive.

Le chargement du catalyseur se fait généralement dans la zone catalytique par tout moyen connu de l'homme du métier, tel un trou d'homme ou tout moyen de distribution dont l'arrivée est située juste au-dessus de chaque lit catalytique, permettant un chargement de préférence étanche du catalyseur (par exemple sous milieu organique). Le déchargement du catalyseur se fait généralement par tout moyen connu de l'homme du métier. Ainsi le déchargement du catalyseur peut se faire par aspiration, par exemple par un trou d'homme, ou par une conduite de vidange.

Les cheminements de la phase liquide et de la phase vapeur à l'intérieur de la zone de distillation réactive, et en particulier à l'intérieur de la zone catalytique, sont caractéristiques d'un des aspects du procédé selon l'invention.

Le dispositif de distillation réactive de l'invention comprend :
a) au moins une zone réactionnelle catalytique renfermant au moins un lit catalytique comportant le catalyseur,
b) au moins une zone de distillation située au-dessous de la zone catalytique précédente, ou zone de distillation inférieure,
c) au moins une autre zone de distillation située au-dessus de la zone catalytique précédente, ou zone de distillation supérieure,

o au moins un moyen de circulation de la majeure partie, de préférence la quasi totalité, du liquide du bas vers le haut à travers le catalyseur de chaque lit catalytique,
o au moins un moyen de circulation de la vapeur du bas vers le haut, de la zone catalytique de telle manière que ladite vapeur ne soit pas en contact avec le catalyseur,
o au moins un moyen de déversement du liquide qui comporte au moins un conduit, de façon à ce que le liquide, après son passage dans le lit catalytique, se déverse dans la zone de distillation inférieure,
o au moins un moyen collecteur,
ledit dispositif étant caractérisé en ce que :
o ledit au moins un moyen collecteur est adapté de façon à ce que le liquide issu de la zone de distillation supérieure arrive en zone centrale de la zone catalytique, au-dessous de chaque lit catalytique de la zone catalytique, ledit moyen collecteur comportant en partie au-dessus de la zone catalytique une zone collectrice et ledit moyen comportant au moins un conduit qui permet la traversée du liquide de haut en bas de la zone catalytique sans avoir de contact avec le catalyseur,
et en ce qu'il comporte :
o au moins un moyen de circulation sensiblement radiale du liquide au-dessous de chaque lit catalytique de la zone catalytique de façon à l'introduire dans au moins une zone de répartition du liquide.

Dans le dispositif selon l'invention, la zone de distillation réactive consiste en une zone réactionnelle catalytique, une zone de distillation inférieure qui est la zone d'épuisement et une zone de distillation supérieure qui est la zone de rectification.

La zone collectrice du moyen collecteur peut permettre le désengagement de la vapeur, c'est-à-dire qu'elle permet de réaliser, par le conduit dudit moyen puis par au moins un moyen de circulation sensiblement radiale du liquide, une alimentation de la zone de répartition correcte, évitant en particulier tout gaz entraîné dans ledit liquide. Ainsi, le liquide se répartit sans turbulences excessives dans ledit conduit puis dans le moyen de circulation sensiblement radiale pour enfin rejoindre la zone de répartition.

La zone de répartition du liquide, alimentée par au moins un moyen de circulation sensiblement radiale, permet la régulation et l'homogénéisation du liquide avant son entrée dans chaque lit catalytique de la zone catalytique. Ainsi un des avantages de la présente invention est d'améliorer la circulation du liquide dans chaque lit catalytique de la zone catalytique Par suite, le procédé est tel que le liquide s'étale sur toute la section de la zone de distillation réactive qui lui est accessible avant d'entrer dans le lit catalytique, dans au moins une zone de répartition du liquide.

De manière préférée, le moyen collecteur est situé en zone centrale de la zone catalytique.

De manière préférée, le liquide qui se déverse dans la zone de distillation inférieure passe en majeure partie en zone périphérique de la zone catalytique. Ainsi, le moyen de déversement comporte au moins un conduit, et tout conduit dudit moyen est situé en zone périphérique de la zone catalytique.

Lorsque la réaction mise en jeu comporte la présence d'au moins un réactif gazeux, le dispositif selon l'invention peut comprendre au moins un moyen d'introduction dudit réactif gazeux, de préférence d'hydrogène, pour chaque lit catalytique de la zone catalytique.

Dans un premier mode de réalisation préféré du dispositif selon l'invention, le moyen collecteur est situé en zone centrale de la zone catalytique, le moyen de déversement est situé en zone périphérique de la zone catalytique, et le moyen de circulation de la vapeur issue de la zone de distillation inférieure est situé en zone centrale de la zone catalytique. De préférence, le moyen de circulation de la vapeur issue de la zone de distillation inférieure comporte au moins un conduit, tout conduit dudit moyen de circulation étant distinct de tout conduit du moyen collecteur de liquide. Dans ce cas, de préférence, le moyen de circulation sensiblement radiale est un dispositif de type "araignée" comportant au moins un bras de distribution, de préférence de 2 à 10 bras de distribution, de manière encore plus préférée de 2 à 8 bras de distribution.

Dans le premier mode de réalisation préféré du dispositif selon l'invention, de manière préférée, le dispositif est tel que le moyen de déversement du liquide est tel qu'il ne permet pratiquement pas de passage de phase vapeur dans le sens ascendant, c'est-à-dire que tout conduit dudit moyen de déversement du liquide comprend au moins un moyen ne permettant pratiquement pas de passage de vapeur dans le sens ascendant.

De manière préférée, dans ledit mode de réalisation, le moyen de circulation de la phase vapeur issue de la zone de distillation inférieure comporte au moins un conduit, et tout conduit dudit moyen est distinct à la fois de tout conduit du moyen collecteur et de tout conduit du moyen de déversement.

Dans un second mode de réalisation préféré du dispositif selon l'invention, le moyen collecteur est situé en zone centrale de la zone catalytique, le moyen de déversement est situé en zone périphérique de la zone catalytique, et le moyen de circulation de la vapeur issue de la zone de distillation inférieure est situé en zone périphérique de la zone catalytique. Alors, un premier mode de réalisation qui est donc un troisième mode de réalisation préféré du dispositif selon l'invention est tel que la vapeur issue de la zone de distillation inférieure et qui circule en zone périphérique de la zone catalytique est au moins en partie, de préférence en majeure partie, en contact avec le liquide circulant dans le moyen de déversement. Ainsi, le moyen de circulation de la vapeur comporte au moins un conduit, et au moins un conduit dudit moyen est aussi un conduit du moyen de déversement du liquide, et de préférence tout conduit dudit moyen est aussi conduit du moyen de déversement du liquide. Et un second mode de réalisation qui est donc un quatrième mode de réalisation préféré du dispositif selon l'invention est tel la vapeur issue de la zone de distillation inférieure et qui circule en zone périphérique de la zone catalytique n'a sensiblement pas de contact avec le liquide circulant dans le moyen de déversement. Ainsi, le moyen de circulation de la vapeur comporte au moins un conduit, tout conduit dudit moyen de circulation étant distinct de tout conduit du moyen de déversement.

De manière préférée, quel que soit le mode de réalisation du dispositif selon l'invention, le moyen de circulation de liquide du bas vers le haut à travers le catalyseur comporte au moins un moyen d'injection dudit liquide dans chaque lit catalytique de la zone catalytique, qui est de préférence une busette. Dans un tel cas, quand la réaction à promouvoir est une réaction comportant au moins une introduction de réactif gazeux qui est l'hydrogène, c'est-à-dire que la réaction comporte une hydrogénation, il est donc généralement nécessaire d'adjoindre un dispositif d'introduction d'hydrogène, pour tout lit catalytique de la zone catalytique, par exemple selon l'une des trois techniques décrites ci-après. Ainsi, la zone catalytique comporte au moins un dispositif d'injection de liquide et au moins un dispositif d'introduction de l'hydrogène dans tout lit catalytique de la zone catalytique. Selon une première technique, le dispositif d'introduction de l'hydrogène est disposé avant le dispositif d'injection du liquide, et donc avant le lit catalytique. Selon une deuxième technique, le dispositif d'introduction de l'hydrogène est disposé au niveau du dispositif d'injection de liquide, de telle façon que l'hydrogène soit introduit dans le liquide avant le lit catalytique. Selon une troisième technique, le dispositif d'introduction de l'hydrogène est disposé après le dispositif d'injection de liquide, et donc au sein du lit catalytique, de préférence non loin dudit dispositif d'injection du liquide dans ledit lit catalytique. Les termes "avant" et "après" utilisé ci-avant s'entendent par rapport au sens de circulation du liquide qui va traverser le lit catalytique.

Quel que soit le moyen de réalisation du dispositif selon l'invention, le moyen de circulation sensiblement radiale peut comporter au moins un bras de distribution, de préférence de 2 à 10 bras de distribution, de manière encore plus préférée de 2 à 8 bras de distribution.

Bien entendu, la vapeur produite lors de la réaction catalytique dans chaque lit catalytique de la zone catalytique rejoint au-dessus dudit lit catalytique la vapeur issue de la zone de distillation inférieure qui traverse ledit lit sans contact avec le catalyseur, quel que soit le mode de réalisation.

Tout autre mode de réalisation du dispositif selon l'invention, combinant les différents modes de réalisation préférés du dispositif selon l'invention, est aussi envisageable. Par exemple il est possible de combiner le premier et le troisième modes de réalisation préférés décrit précédemment, c'est-à-dire qu'une partie de la vapeur issue de la zone de distillation inférieure circule dans au moins un conduit en zone périphérique de la zone catalytique et que l'autre partie de ladite vapeur circule dans au moins un conduit en zone centrale de la zone catalytique.

Le dispositif de distillation réactive selon l'invention consiste en au moins une colonne catalytique. Dans les cas connus de l'homme du métier où la mise en oeuvre d'une seule colonne pose des problèmes, on préfère généralement scinder ladite colonne de façon à utiliser finalement au moins deux colonnes qui, mises bout à bout, réalisent ladite colonne, c'est-à-dire que les zones de rectification (ou zone supérieure), de distillation catalytique et d'épuisement (ou zone inférieure) se répartissent sur les colonnes. En pratique, la zone de rectification ou la zone d'épuisement, et de préférence la zone d'épuisement, peut se trouver généralement dans au moins une colonne différente de la colonne comprenant la zone catalytique.

La zone de rectification du dispositif selon l'invention comporte généralement soit au moins un plateau distributeur éventuellement situé au-dessus d'une zone comportant au moins un garnissage assurant la distillation, soit au moins un plateau de distillation, qui peut être conventionnel, ainsi que toute combinaison de garnissage(s), de plateau(x) distributeur(s) et de plateau(x) de distillation assurant la distillation selon toute technique connue de l'homme du métier.

De même, la zone d'épuisement du dispositif selon l'invention comporte généralement soit au moins un plateau distributeur éventuellement situé au-dessus d'une zone comportant au moins un garnissage assurant la distillation, soit au moins un plateau de distillation, qui peut être conventionnel, ainsi que toute combinaison de garnissage(s), de plateau(x) distributeur(s) et de plateau(x) de distillation assurant la distillation selon toute technique connue de l'homme du métier.

Les caractéristiques du plateau distributeur, du corps de garnissage, du catalyseur et du chargement et du déchargement du catalyseur, pour le dispositif selon l'invention, sont les mêmes que celles données précédemment dans le cas du procédé selon l'invention.

Le dispositif de distillation réactive selon l'invention peut comprendre au moins un moyen de collecte d'une partie du liquide s'écoulant par exemple du lit catalytique inférieur de la zone catalytique située le plus bas dans la zone catalytique sur la zone de distillation inférieure, au moins un moyen de filtrage (situé généralement à l'extérieur de la zone de distillation réactive), qui permet de retenir les fragments éventuels des particules catalytiques constituant le catalyseur éventuellement entraînées par la phase liquide, et au moins un moyen de redistribution du liquide (sensiblement exempt de fragments de particules catalytiques) dans la zone catalytique, généralement entre la position d'où il a été soutiré et la zone de distillation inférieure.

Les cheminements de la phase liquide et de la phase vapeur à l'intérieur de ladite zone de distillation réactive, et en particulier à l'intérieur de la zone catalytique, sont imposés par les internes de la zone de distillation réactive et sont caractéristiques d'un des aspects du dispositif selon l'invention.

Par ailleurs, dans le cas de réactions exothermiques, par exemple pour la synthèse des éthers alkyliques tertiaires, un des avantages du procédé et du dispositif selon l'invention est que la circulation en courant ascendant de la phase liquide à travers les lits catalytiques permet en outre d'évacuer aisément la phase vapeur générée sur le catalyseur par la chaleur de réaction.

Le procédé et le dispositif de l'invention sont illustrés par les figures ci-jointes. La figure 1 illustre sous une forme schématique l'agencement général de ladite zone ; les figures 2, 2A, 2B et 2C illustrent la configuration de zones de distillation et de zones catalytiques qui composent au moins en partie ladite zone, selon le premier mode de réalisation préféré du dispositif selon l'invention ; les figures 3, 4, 4A et 4C illustrent la configuration de zones de distillation et de zones catalytiques qui composent au moins en partie ladite zone, selon le troisième mode de réalisation préféré du dispositif selon l'invention ; les figures 5 et 5A illustrent la configuration de zones de distillation et de zones catalytiques qui composent au moins en partie ladite zone, selon le quatrième mode de réalisation préféré du dispositif selon l'invention. Les mêmes numéros sont utilisés pour représenter les organes similaires sur toutes les figures.

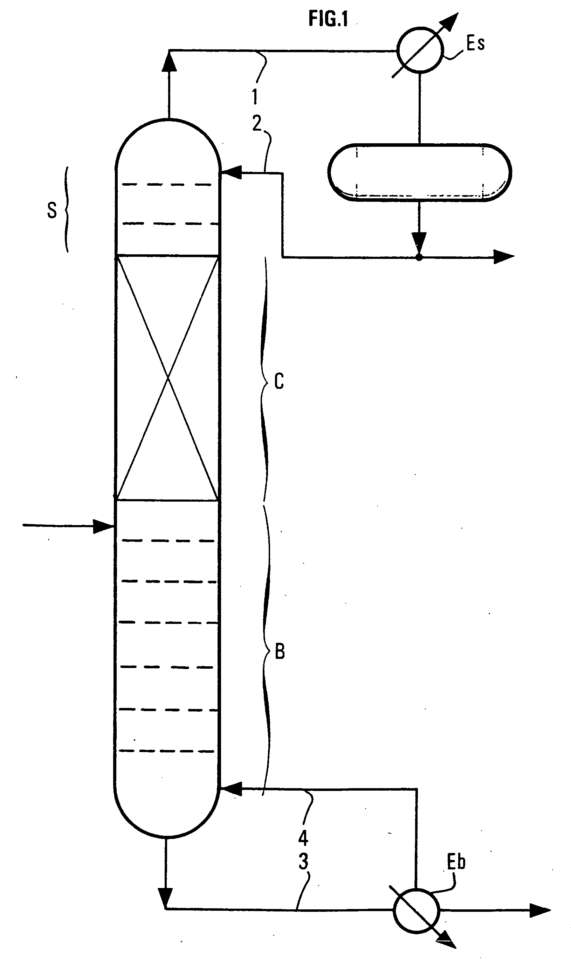

La zone de distillation réactive utilisée dans le procédé de l'invention qui est représentée sur la figure 1 est une colonne catalytique qui comprend essentiellement trois zones, à savoir : une zone catalytique (C), dans laquelle progressent la distillation et la réaction chimique, l'une favorisant l'autre, un sommet (S) (ou zone de rectification) et une base (B) (ou zone d'épuisement).

Le sommet (S) est équipé d'une ligne 1 pour l'évacuation des vapeurs des constituants les plus volatils et d'une ligne 2 pour l'introduction d'un reflux liquide constitué par une fraction desdites vapeurs condensées dans l'échangeur Es.

La base (B) est équipée d'une ligne 3 pour l'évacuation des constituants les moins volatils sous forme liquide et d'une ligne 4 pour l'introduction de la vapeur de rebouillage générée par vaporisation partielle d'au moins une partie desdits constituants dans l'échangeur Eb. Elle est également équipée d'une ou plusieurs conduites pour l'introduction éventuelle de tout ou partie des réactifs.

La zone catalytique (C) reçoit, de manière interne, une phase liquide générée par le reflux introduit au sommet de la zone de distillation réactive et une phase vapeur générée par la vapeur de rebouillage introduite à la base de la zone de distillation réactive ainsi que, de manière externe, à au moins un niveau, un apport éventuel d'au moins un des réactifs, pur ou dilué par exemple d'alcool dans le cas de l'utilisation du dispositif en synthèse d'éthers. Dans la figure 1, on n'a pas représenté une telle introduction.

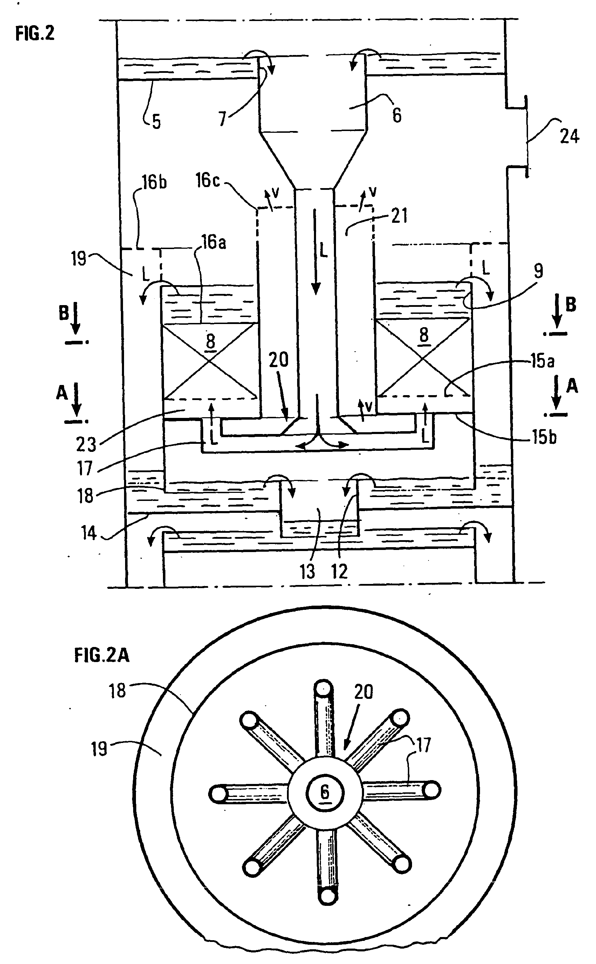

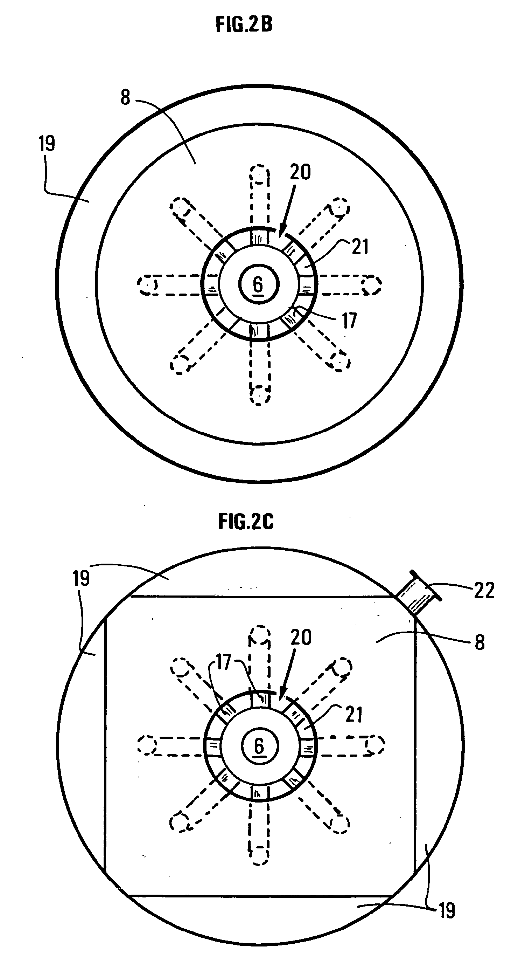

Une disposition du lit catalytique de la zone catalytique, associé à deux zones de distillation et formant une des caractéristiques du dispositif selon l'invention, est décrit ci-après en liaison avec les figures 2, 2A, 2B et 2C, qui en illustrent deux formes de réalisation dans le cas du premier mode de réalisation préféré du dispositif selon l'invention. La zone catalytique comprend un seul lit catalytique 8 et est bordée en sa partie supérieure par un plateau de distillation 5 et en sa partie inférieure par un plateau de distillation 14. Le plateau 5 de distillation, qui comporte un déversoir 7 central, reliant ledit plateau à une descente 6, est situé au-dessus du lit catalytique et est soit le plateau le plus bas de la zone (S), soit l'unique plateau de la zone (S) si celle-ci ne comprend qu'un seul plateau, soit le plateau le plus bas d'une zone de distillation comprise dans la zone catalytique (C), soit l'unique plateau de ladite zone comprise dans la zone (C) si celle-ci ne comprend qu'un seul plateau. Le plateau 14 de distillation, qui comporte un déversoir 12 central, reliant ledit plateau à une descente 13, est situé au-dessous du lit catalytique et est soit le plateau le plus haut de la zone (B), soit l'unique plateau de la zone (B) si celle-ci ne comprend qu'un seul plateau, soit le plateau le plus haut d'une zone de distillation comprise dans la zone de distillation catalytique (C), soit l'unique plateau de ladite zone comprise dans la zone (C) si celle-ci ne comprend qu'un seul plateau.

Le lit catalytique 8 est adapté pour être traversé de bas en haut par la phase liquide sans être traversé par la phase vapeur issue de la zone de distillation.

Le catalyseur à installer dans l'ensemble des lits catalytiques 8 est conditionné sous toute forme adéquate, notamment sous forme de particules catalytiques de forme sensiblement cylindrique ou de forme sensiblement sphérique, les dimensions des particules de catalyseur étant comprises entre 0,1 et 20 mm. Pour maintenir le catalyseur en place et pour éviter qu'il soit entraîné par le courant de liquide qui le traverse, le lit catalytique repose sur une grille inférieure 15a. Avantageusement, la grille inférieure 15a est légèrement surélevée par rapport à l'arrivée des moyens de distribution de liquide 17, sur le fond (plaque 15b) de la zone catalytique, de manière à créer une zone de répartition de la phase liquide 23, dans laquelle arrive la phase liquide issue du moyen de circulation sensiblement radiale du liquide au-dessous de la zone catalytique, avant que la phase liquide ne circule dans le lit catalytique.

Il est possible aussi de prévoir la présence d'une grille supérieure 16a imperméable au passage des particules catalytiques, de façon à confiner lesdites particules dans le lit catalytique. De façon alternative, il est possible de prévoir tout moyen tels que les moyens 16b ou 16c, illustrés ci-après sur la figure 2, de façon à éviter que le catalyseur ne déborde du lit catalytique lors du fonctionnement. Il est aussi possible de combiner des moyens tels que ceux des références 16a, 16b ou 16c illustrés sur la figure 2.

Plus particulièrement, le cas du premier mode de réalisation préféré du dispositif selon l'invention est représenté aux figures 2, 2A, 2B et 2C, les figures 2B et 2C représentant chacune une possibilité de coupe de la figure 2 suivant l'axe BB, et la figure 2A représentant une coupe de la figure 2 suivant l'axe AA. Le lit catalytique 8 reçoit la phase liquide s'écoulant du plateau de distillation 5 par débordement par-dessus le déversoir central 7 et par la descente 6. L'alimentation du lit catalytique en liquide de distillation est une des caractéristiques de la présente invention. Il se fait par l'intermédiaire d'un moyen de circulation sensiblement radiale 20 du liquide comportant huit bras d'alimentation 17, qui permet le passage ascendant de la phase vapeur dans la conduite 21 de la zone de distillation inférieure à la zone de distillation supérieure sans contact avec le liquide. La figure 2A permet de bien comprendre une structure particulière possible dudit moyen 20 comportant les bras de distribution 17, les références 18 et 19 étant explicitées ci-après.

A travers le lit catalytique 8, la phase liquide se déplace du bas vers le haut et se déverse sur le plateau de distillation 14 par débordement par dessus le déversoir 9 et écoulement par la descente 19. Il est possible dans le cadre de la présente invention de mettre, au-dessus du déversoir 9, tout moyen tel que le moyen 16b illustré sur la figure 2, perméable au liquide et imperméable aux particules catalytiques, par exempte une grille de type Johnson. Le plateau 14 présente une descente centrale 13 et un déversoir 12. De préférence, comme représenté sur les figures 2 et 2A, la descente 19 est telle qu'elle comprend un moyen 18 ne permettant pratiquement pas de passage de phase vapeur dans le sens ascendant dans ladite descente 19. Dans ce cas, le plateau 14 ne comprend bien évidemment pas de moyen de contact liquide-vapeur, susceptible de générer de la vapeur de distillation, au-dessous de ladite descente 19.

D'autre part, la phase vapeur générée par la zone de distillation inférieure circule dans le sens ascendant en zone centrale de la zone catalytique par la conduite 21, son passage vers la conduite 21 étant possible grâce à la structure du moyen de distribution 20 (voir en particulier les figures 2A, 2B et 2C). Il est possible dans le cadre de la présente invention de mettre en sortie de la conduite 21, ladite sortie se situant au-dessus du lit catalytique, tout moyen tel que le moyen 16c, illustré sur la figure 2, perméable à la vapeur et imperméable aux particules catalytiques, par exemple une grille de type Johnson.

Le chargement du catalyseur se fait généralement dans la zone de distillation réactive par tout moyen connu de l'homme du métier, tel le trou d'homme 24 illustré sur la figure 2. Quant au déchargement, plusieurs configurations sont possibles dans le cadre du premier mode de réalisation préféré du dispositif selon l'invention, parmi lesquelles deux possibilités ont été illustrées plus particulièrement sur les figures 2B et 2C qui présentent toutes deux une possibilité de coupe de la figure 2 suivant l'axe BB. Ainsi le déchargement du catalyseur peut se faire par aspiration, par exemple par le trou d'homme 24 lorsque la configuration est celle de la figure 2B, ou par une conduite de vidange 22 spécialement aménagée à cet effet lorsque la configuration est celle de la figure 2C.

Le procédé et le dispositif de l'invention présentent notamment l'avantage de bien contrôler la circulation de liquide à l'intérieur des zones catalytiques. En particulier, il est préféré dans le cadre de la présente invention que toute section de zone catalytique soit telle que le liquide circulant dans le lit catalytique au niveau de ladite section puisse librement se répartir dans ledit niveau, c'est-à-dire que toutes les particules catalytiques constituant le catalyseur soient en contact au niveau d'une même section. De plus, l'existence d'une zone de répartition 23 (figure 2) permet d'améliorer notablement l'irrigation du lit catalytique. En outre, la mise en oeuvre d'un courant ascendant de phase liquide à travers les lits de catalyseur amène une expansion et une certaine mobilité de ces lits, ce qui permet d'éviter de colmater les lits catalytiques. Enfin, la descente 6 du liquide est optimisée de façon à permettre une arrivée homogène du liquide descendant avant sa distribution par le moyen 20.

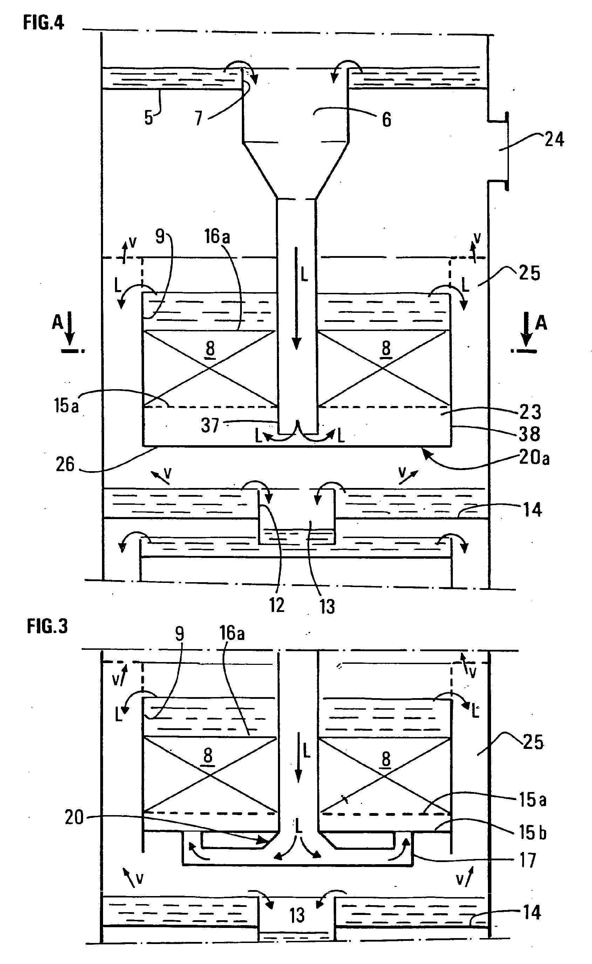

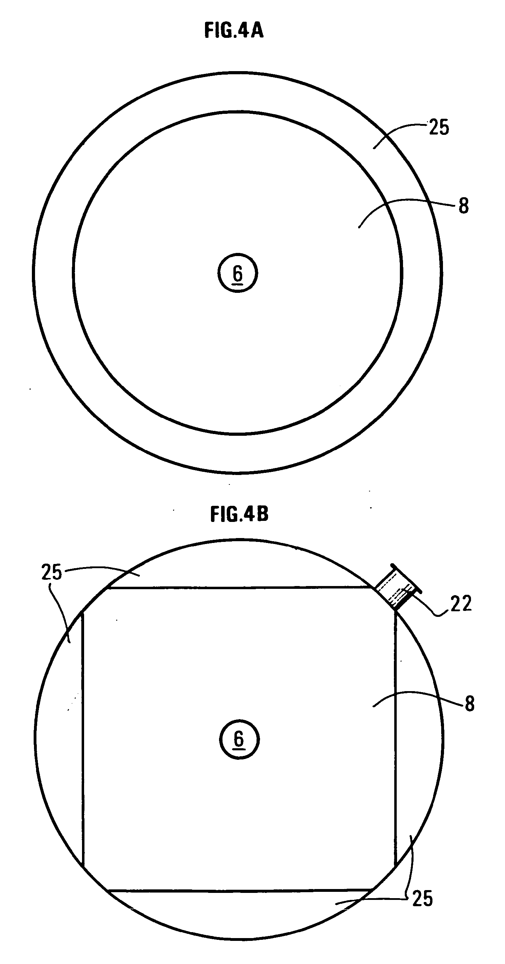

Les figures 3, 4, 4A, et 4B permettent d'illustrer deux formes de réalisation dans le cas du troisième mode de réalisation préféré du dispositif selon l'invention. Les références de la figure 2 s'appliquent aux figures 3 et 4, mais dans ce cas la vapeur circule en majeure partie en zone périphérique, en remontant par la descente 25 par laquelle s'écoule le liquide qui est passé à travers le lit catalytique 8, par déversement par dessus le déversoir 9. Dans ce cas, le moyen 20a de circulation sensiblement radiale du liquide prend une forme sensiblement différente du moyen 20 illustré sur la figure 2. Pour la figure 4, il comprend essentiellement le tube 37 et il est associé à la forme cylindrique 38 et à une plaque 15b qui maintient le catalyseur, de façon à créer la zone de répartition 23. Contrairement au cas de la figure 2, la plaque 15b a été omise, le fond de la zone catalytique étant constitué de la plaque 26. Mais ce n'est qu'une possibilité. Parmi d'autres possibilités, le dispositif (représenté sur la figure 3) est composé du moyen 20 de la figure 2 avec des bras d'alimentation 17 associé à une plaque 26.

Les figures 4A et 4B présentent deux possibilités de coupe de la figure 4 suivant l'axe AA. Le déchargement du catalyseur peut se faire par aspiration, par exemple par le trou d'homme 24 lorsque la configuration est celle de la figure 4A, ou par une conduite de vidange 22 lorsque la configuration est celle de la figure 4B.

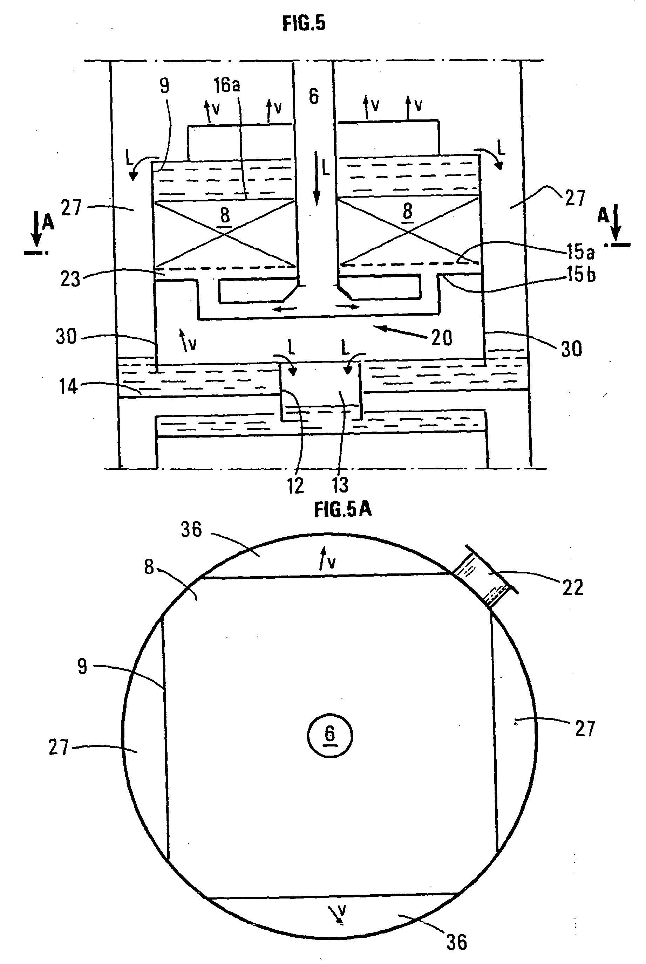

Les figures 5 et 5A permettent d'illustrer une forme de réalisation dans le cas du quatrième mode de réalisation préféré du dispositif selon l'invention. Les références de la figure 2 s'appliquent à la figure 5, comme il a été expliqué ci-dessus, mais dans ce cas la vapeur circule en majeure partie en zone périphérique, en remontant par deux espaces 36 tandis que le liquide qui est passé à travers le lit catalytique 8 se déverse par dessus deux déversoirs 9 dans deux descentes 27. Dans ce cas, le moyen de circulation sensiblement radiale du liquide 20a est sensiblement le même que celui décrit ci-dessus dans le cas du troisième mode de réalisation préféré du dispositif selon l'invention illustré par la figure 3. La figure 5A présente une coupe de la figure 5 suivant l'axe AA. Le déchargement du catalyseur se fait par une conduite de vidange 22. Bien sûr, dans ce cas, le dispositif est muni de tout moyen adéquat pour empêcher sensiblement totalement la remontée de vapeur par les descentes de liquide 27, et pour empêcher le débordement d'une partie du liquide qui a traversé le lit catalytique 8 dans les espaces 36.

Comme il a été exposé précédemment, le procédé et le dispositif de l'invention peuvent s'appliquer à diverses réactions équilibrées, en phase liquide, pour lesquelles on peut isoler le produit de réaction par distillation dans les conditions de température et de pression auxquelles on réalise la réaction, et notamment les réactions d'alkylation d'hydrocarbures aromatiques avec l'oléfine appropriée, les réactions d'isomérisation de paraffines, les réactions d'isomérisation d'oléfines ou la production de butène-2 par hydroisomérisation, De façon préférée, ils s'appliquent plus particulièrement aux réactions d'éthérification entre une isooléfine (par exemple l'isobutène ou l'isopentène) et un monoalcool aliphatique (par exemple le méthanol ou l'éthanol) pour former les éthers correspondants. Les conditions de mise en oeuvre de ces réactions sont bien connues de l'homme du métier. Cependant à titre indicatif on donne ci-après les conditions de synthèse du méthyltertiobutyléther.

Les conditions habituellement mises en oeuvre sont généralement une pression comprise entre 0,4 et 1,6 MPa, une température de fond de zone de distillation réactive, fonction de la pression choisie, comprise entre 110 et 170 °C et une température de tête de zone de distillation réactive, fonction de la pression choisie, comprise entre 40 et 90 °C. On maintient en général un taux de reflux par rapport au distillat compris entre 0,5:1 et 5:1. Par ce procédé il est possible de convertir la quasi totalité de l'isobutène et d'obtenir du MTBE avec une pureté élevée, en général d'au moins 98% en moles. La charge contenant de l'isobutène est en général constituée d'une coupe C₄ (charge hydrocarbonée comprenant en majeure partie des composés ayant 4 atomes de carbone par molécule), provenant notamment d'une unité de vapocraquage, d'un craquage catalytique ou de la déshydrogénation de l'isobutane. Dans une première zone de réaction, elle est mise en contact avec du méthanol, dans des conditions réactionnelles bien connues de l'homme du métier. Cette réaction, équilibrée, permet de convertir une partie de l'isobutène (en général de 70 à 90 %) en MTBE. C'est le mélange issu de cette première zone qui est ensuite traité selon le procédé de distillation réactive de l'invention ou dans le dispositif de distillation réactive selon l'invention. La charge contenant de l'isobutène et du méthanol non convertis est généralement introduite juste au-dessous de la zone catalytique. Un appoint de méthanol est généralement introduit juste au-dessus de ladite zone. On peut également prévoir une introduction de cet appoint en plusieurs points répartis au long de la zone catalytique. Le catalyseur est placé dans les zones catalytiques de la zone de distillation réactive comme décrit plus haut.

Les exemples suivants illustrent l'invention.

### EXEMPLE 1

Dans cet exemple, réalisé selon le premier mode de réalisation préféré du procédé de l'invention, on traite une charge issue d'une première section réactionnelle d'éthérification où l'isobutène a été converti à 88 %. La composition de l'alimentation de la colonne de distillation réactive, selon l'invention, est donnée dans la troisième colonne du tableau 1. Elle tient compte d'un appoint d'alcool supplémentaire fourni à la colonne, représentant 0,5 pour 100 en poids.

**TABLEAU 1**

| | Charge unité | Charge colonne catalytique | Fond de colonne (% poids) | Tête de colonne (%poids) |
|---|---|---|---|---|
| Isobutène | 22,5 | 2,70 | < 0,002 | 0,62 |
| n-butane | 7,0 | 7,0 | < 0,01 | 10,64 |
| butènes | 39,0 | 39,0 | <0,01 | 59,28 |
| isobutane | 18,0 | 18,0 | < 0,01 | 27,36 |
| méthanol | 13,5 | 2,69 ** | - | 2,10 |
| MTBE | | 31,11 | 99,2 | < 0,02 |
| TOTAL (g) | 100 | 100,5 | | |
| | | | | |
| Conversion de l'isobutène | | 88 % | | 98,2 % |

| | | | | |
|---|---|---|---|---|
| ** un appoint supplémentaire de méthanol (0,5 pour 100) est fournie à la colonne. | | | | |

On utilise une colonne de hauteur 350 cm et de diamètre 5 cm. Elle présente une zone supérieure (zone de rectification) équipée de 8 plateaux de distillation de type double passe et une zone inférieure (zone d'épuisement) équipée de 15 plateaux de distillation de type double passe. La zone catalytique est constituée d'une alternance de zone de distillation et de zone de réaction, selon le premier mode de réalisation préféré du dispositif de l'invention. (voir figures 2, 2A, 2B et 2C). Dans cette zone catalytique, il y a cinq lits catalytiques, contenant chacun 6 cm3 de catalyseur (résine sulfonique, vendue par Rohm & Haas sous la marque déposée Amberlyst 15), séparés des uns des autres par trois plateaux de distillation de type double passe.

La colonne est opérée sous une pression relative de 0,7 MPa, avec un taux de reflux de 1 : 1.

La charge est alimentée au niveau du vingt-sixième plateau (compté de haut en bas). L'appoint de méthanol est injecté sur le plateau juste au dessus du premier lit catalytique (c'est-à-dire sur le plateau 8). Après mise en régime, il s'établit dans la colonne un profil de température allant de 62 °C en tête à 139°C en fond.

Pour une alimentation de la colonne de 1800 g/h (plus 9 g/h de méthanol), on sort en tête un distillat ayant la composition donnée dans le tableau 2 avec un débit de 1183 g/h. On sort en fond 623 g/h d'un produit MTBE d'une pureté supérieure à 99 %. La conversion de l'isobutène dans la colonne s'élève à 85 %.

### EXEMPLE 2

Dans cet exemple, réalisé selon le troisième mode de réalisation préféré du procédé de l'invention, on traite une charge issue d'une première section réactionnelle d'éthérification où l'isobutène a été converti à 88 %. La composition de l'alimentation de la colonne de distillation catalytique, selon l'invention, est donnée dans la troisième colonne du tableau 2. Elle tient compte d'un appoint d'alcool supplémentaire fournie à la colonne, représentant 0,5 pour 100 en poids.

**TABLEAU 2**

| | Charge unité | Charge colonne catalytique | Fond de colonne (% poids) | Tête de colonne (% poids) |
|---|---|---|---|---|
| Isobutène | 22,5 | 2,70 | < 0,002 | 0,66 |
| n-butane | 7,0 | 7,0 | < 0,01 | 10,63 |
| butènes | 39,0 | 39,0 | < 0,01 | 59,24 |
| isobutane | 18,0 | 18,0 | < 0,01 | 27,34 |
| méthanol | 13,5 | 2,69** | - | 2,12 |
| MTBE | | 31,11 | 99,2 | < 0,02 |
| TOTAL (g) | 100 | 100,5 | | |
| | | | | |
| Cv. de l'isobutène | | 88 % | | 98,1 % |

| | | | | |
|---|---|---|---|---|
| ** un appoint supplémentaire de méthanol (0,5 pour 100) est fournie à la colonne. | | | | |

On utilise une colonne de hauteur 350 cm et de diamètre 5 cm. Elle présente une zone supérieure (zone de rectification) équipée de garnissage Sulzer et une zone inférieure (zone d'épuisement) équipée également de garnissage Sulzer. La zone catalytique est constituée d'une alternance de zone de distillation et de zone de réaction, selon le troisième mode de réalisation préféré du dispositif de l'invention. (voir figures 4 et 4A). Dans cette zone catalytique, il y a cinq lits catalytiques, contenant chacun 6 cm3 de catalyseur (résine sulfonique, vendue par Rohm & Haas sous la marque déposée Amberlyst 15), séparés des uns des autres par trois plateaux de distillation de type double passe.

La colonne est opérée sous une pression relative de 0,7 MPa, avec un taux de reflux de 1 : 1.

La charge est alimentée au niveau du vingt-sixième plateau (compté de haut en bas). L'appoint de méthanol est injecté sur le plateau juste au dessus du premier lit catalytique (c'est-à-dire le plateau 8). Après mise en régime, il s'établit dans la colonne un profil de température allant de 62 °C en tête à 139 °C en fond.

Pour une alimentation de la colonne de 1800 g/h (plus 9 g/h de méthanol),- on sort en tête un distillat ayant la composition donnée dans le tableau 1 avec un débit de 1182 g/h. On sort en fond 622 g/h d'un produit MTBE d'une pureté supérieure à 99 %. La conversion de l'isobutène dans la colonne s'élève à 84 %.

### EXEMPLE 3

Dans cet exemple, réalisé selon le quatrième mode de réalisation préféré du procédé selon l'invention, on traite une charge issue d'une première section réactionnelle d'éthérification où l'isobutène a été converti à 88 %. La composition de l'alimentation de la colonne de distillation catalytique, selon l'invention, est donnée dans la troisième colonne du tableau 3. Elle tient compte d'un appoint d'alcool supplémentaire fournie à la colonne, représentant 0,5 pour 100 en poids.

**TABLEAU 3**

| | Charge unité | Charge colonne catalytique | Fond de colonne (% poids) | Tête de colonne (% poids) |
|---|---|---|---|---|
| Isobutène | 22,5 | 2,70 | < 0,002 | 0,57 |
| n-butane | 7,0 | 7,0 | < 0,01 | 10,65 |
| butènes | 39,0 | 39,0 | < 0,01 | 59,32 |
| isobutane | 18,0 | 18,0 | < 0,01 | 27,38 |
| méthanol | 13,5 | 2,69 ** | - | 2,08 |
| MTBE | | 31,11 | 99,1 | <0,02 |
| TOTAL (g) | 100 | 100,5 | | |
| | | | | |
| Cv. de l'isobutène | | 88 % | | 98,3 % |

| | | | | |
|---|---|---|---|---|
| ** un appoint supplémentaire de méthanol (0,5 pour 100) est fournie à la colonne. | | | | |

On utilise une colonne de hauteur 350 cm et de diamètre 5 cm. Elle présente une zone supérieure (zone de rectification) équipée de garnissage Sulzer et une zone inférieure (zone d'épuisement) équipée également de garnissage Sulzer. La zone catalytique est constituée d'une alternance de zone de distillation et de zone de réaction, selon le quatrième mode de réalisation du dispositif de l'invention. (voir figures 5 et 5A). Dans cette zone catalytique, il y a cinq lits catalytiques, contenant chacun 6 cm3 de catalyseur (résine sulfonique, vendue par Rohm & Haas sous la marque déposée Amberlyst 15), séparés des uns des autres par trois plateaux de distillation de type double passe.

La colonne est opérée sous une pression relative de 0,7 MPa, avec un taux de reflux de 1 : 1.

La charge est alimentée au niveau du vingt-sixième plateau (compté de haut en bas). L'appoint de méthanol est injecté sur le plateau juste au dessus du premier lit catalytique (c'est-à-dire le plateau 8). Après mise en régime, il s'établit dans la colonne un profil de température allant de 62 °C en tête à 139 °C en fond.

Pour une alimentation de la colonne de 1800 g/h (plus 9 g/h de méthanol), on sort en tête un distillat ayant la composition donnée dans le tableau 3 avec un débit de 1183 g/h. On sort en fond 623 g/h d'un produit MTBE d'une pureté supérieure à 99 %. La conversion de l'isobutène dans la colonne s'élève à 86 %.

## Revendications

1. Procédé de distillation réactive tel que :
• on introduit des réactifs, seuls ou dilués, l'un des réactifs pouvant être excédentaire, en au moins un niveau d'une zone de distillation réactive qui renferme :
a) au moins une zone réactionnelle catalytique renfermant au moins un lit catalytique comportant le catalyseur,
b) au moins une zone de distillation située au-dessous de la zone catalytique précédente, ou zone de distillation inférieure,
c) au moins une autre zone de distillation située au-dessus de la zone catalytique précédente, ou zone de distillation supérieure,
• on maintient des conditions de distillation de façon à avoir une phase liquide et une phase vapeur dans la zone de distillation réactive,
• on fait circuler la majeure partie du liquide du bas vers le haut à travers le catalyseur dans la zone catalytique,
• on fait circuler la majeure partie de la vapeur du bas vers le haut dans la zone catalytique, de telle manière que ladite vapeur ne soit pas en contact avec le catalyseur,
• on recueille une partie du produit de réaction recherché,
• on recueille une partie d'un diluant éventuel et des réactifs,
• on récupère la majeure partie du liquide après son passage dans le lit catalytique par au moins un moyen de déversement du liquide, de façon à ce que le liquide se déverse dans la zone de distillation inférieure,
• et on collecte la majeure partie du liquide issu de la zone de distillation supérieure,
ledit procédé étant **caractérisé en ce que** :
• on collecte la majeure partie du liquide issu de la zone de distillation supérieure par au moins un moyen collecteur de façon à ce que le liquide arrive en zone centrale de la zone catalytique, au-dessous de chaque lit catalytique de la zone catalytique, sans avoir de contact avec le catalyseur,
• on fait circuler sensiblement radialement la majeure partie du liquide au-dessous dudit lit catalytique par au moins un moyen de circulation sensiblement radiale de façon à l'introduire dans au moins une zone de répartition du liquide.

2. Procédé selon la revendication 1 comportant au moins l'introduction d'un réactif gazeux pour chaque lit catalytique de la zone catalytique.

3. Procédé selon l'une des revendications 1 et 2 tel que le liquide qui se déverse dans la zone de distillation inférieure par au moins un moyen de déversement passe en majeure partie en zone périphérique de la zone catalytique.

4. Procédé selon l'une des revendications 1 à 3 tel que le liquide issu de la zone de distillation supérieure qui est collecté circule en zone centrale de la zone catalytique, le liquide qui se déverse dans la zone de distillation inférieure passe en majeure partie en zone périphérique de la zone catalytique, et la vapeur issue de la zone de distillation inférieure et qui circule dans la zone catalytique circule en zone centrale de la zone catalytique.

5. Procédé selon l'une des revendications 1 à 4 tel que le liquide issu de la zone de distillation supérieure qui est collecté circule en zone centrale de la zone catalytique, le liquide qui se déverse dans la zone de distillation inférieure passe en majeure partie en zone périphérique de la zone catalytique, et la vapeur issue de la zone de distillation inférieure et qui circule dans la zone catalytique circule en zone périphérique de la zone catalytique.

6. Procédé selon la revendication 5 tel que la vapeur issue de la zone de distillation inférieure et qui circule en zone périphérique de la zone catalytique n'a pas de contact avec le liquide circulant dans le moyen de déversement.

7. Procédé selon l'une des revendications 1 à 6 tel que le moyen de circulation radiale comporte au moins un bras de distribution.

8. Dispositif de distillation réactive comprenant :
a) au moins une zone réactionnelle catalytique renfermant au moins un lit catalytique comportant le catalyseur,
b) au moins une zone de distillation située au-dessous de la zone catalytique précédente, ou zone de distillation inférieure,
c) au moins une autre zone de distillation située au-dessus de la zone catalytique précédente, ou zone de distillation supérieure,
• au moins un moyen de circulation de la majeure partie du liquide du bas vers le haut à travers le catalyseur dans chaque lit catalytique,
• au moins un moyen de circulation de la vapeur du bas vers le haut de la zone catalytique, de telle manière que ladite vapeur ne soit pas en contact avec le catalyseur,
• au moins un moyen de déversement du liquide qui comporte au moins un conduit, de façon à ce que le liquide après son passage dans le lit catalytique se déverse dans la zone de distillation inférieure,
• au moins un moyen collecteur,
ledit dispositif étant **caractérisé en ce que**
• ledit au moins un moyen collecteur est adapté de façon à ce que le liquide issu de la zone de distillation supérieure arrive en zone centrale de la zone catalytique, au-dessous de chaque lit catalytique de la zone catalytique, ledit moyen collecteur comportant, en partie au-dessus de la zone catalytique, une zone collectrice et ledit moyen comportant au moins un conduit qui permet la traversée du liquide de haut en bas de la zone catalytique sans avoir de contact avec le catalyseur,
et **en ce qu'**il comporte :
• au moins un moyen de circulation sensiblement radiale du liquide au-dessous de chaque lit de la zone catalytique de façon à l'introduire dans au moins une zone de répartition du liquide.

9. Dispositif selon la revendication 8 tel que la zone collectrice du moyen collecteur permet le désengagement de la vapeur.

10. Dispositif selon l'une des revendications 8 ou 9 comportant au moins un moyen d'introduction d'un réactif gazeux pour chaque lit catalytique de la zone catalytique.

11. Dispositif selon l'une des revendications 8 à 10 tel que le moyen de déversement du liquide qui se déverse dans la zone de distillation inférieure est situé en zone périphérique de la zone catalytique.

12. Dispositif selon l'une des revendications 8 à 11 tel que le moyen collecteur est situé en zone centrale de la zone catalytique, le moyen de déversement est situé en zone périphérique de la zone catalytique, et le moyen de circulation de la vapeur issue de la zone de distillation inférieure est situé en zone centrale de la zone catalytique.

13. Dispositif selon l'une des revendications 8 à 12 tel que le moyen collecteur est situé en zone centrale de la zone catalytique, le moyen de déversement est situé en zone périphérique de la zone catalytique, et le moyen de circulation de la vapeur issue de la zone de distillation inférieure est situé en zone périphérique de la zone catalytique.

14. Dispositif selon la revendication 13 tel que le moyen de circulation de la vapeur issue de la zone de distillation inférieure comporte au moins un conduit, tout conduit dudit moyen de circulation étant distinct de tout conduit du moyen de déversement du liquide.

15. Dispositif selon l'une des revendications 8 à 14 tel que le moyen de circulation de liquide du bas vers le haut à travers le catalyseur comporte au moins un moyen d'injection dudit liquide dans chaque lit catalytique de la zone catalytique.

16. Dispositif selon la revendication 15 tel que tout lit catalytique de la zone catalytique comporte au moins un dispositif d'introduction d'hydrogène.

17. Dispositif selon la revendication 16 tel que le dispositif d'introduction de l'hydrogène est disposé avant le dispositif d'injection de liquide.

18. Dispositif selon la revendication 16 tel que le dispositif d'introduction de l'hydrogène est disposé au niveau du dispositif d'injection de liquide.

19. Dispositif selon la revendication 16 tel que le dispositif d'introduction de l'hydrogène est disposé après le dispositif d'injection de liquide.

20. Dispositif selon l'une des revendications 8 à 19 tel que le moyen de circulation sensiblement radiale comporte au moins un bras de distribution.

## Claims

1. A reactive distillation method such that :
• reagents are fed, alone or diluted, with possibly one excess reagent, into at least one level of a reactive distillation zone containing:
a) at least one catalytic reaction zone including at least one catalytic bed comprising the catalyst,
b) at least one distillation zone located below the aforementioned catalytic zone, or lower distillation zone,
c) at least another distillation zone located above the aforementioned catalytic zone, or upper distillation zone,
• distillation conditions are maintained so as to have a liquid phase and a vapour phase in the reactive distillation zone,
• the major part of the liquid is circulated from bottom to top through the catalyst in the catalytic zone,
• the major part of the vapour is circulated from bottom to top in the catalytic zone, in such a way that said vapour is not in contact with the catalyst,
• part of the wanted reaction product is collected,
• part of a possible diluent and of the reagents is collected,
• the major part of the liquid is recovered after passage in the catalytic bed through the agency of at least one liquid discharge means so that the liquid flows into the lower distillation zone,
• and the major part of the liquid from the upper distillation zone is collected,
said method being **characterized in that**:
• the major part of the liquid from the upper distillation zone is collected by at least one collecting means so that the liquid reaches the central zone of the catalytic zone, below each catalytic bed of the catalytic zone, without being in contact with the catalyst,
• the major part of the liquid is circulated substantially radially below said catalytic bed through the agency of at least one substantially radial circulation means so as to feed it into at least one liquid distribution zone.

2. A method as claimed in claim 1, comprising feeding at least one gaseous reagent for each catalytic bed of the catalytic zone.

3. A method as claimed in any one of claims 1 and 2, such that the major part of the liquid flowing into the lower distillation zone through at least one discharge means passes into the peripheral zone of the catalytic zone.

4. A method as claimed in any one of claims 1 to 3, such that the collected liquid from the upper distillation zone circulates in the central zone of the catalytic zone, the major part of the liquid flowing into the lower distillation zone passes into the peripheral zone of the catalytic zone, and the vapour from the lower distillation zone circulating in the catalytic zone circulates in the central zone of the catalytic zone.

5. A method as claimed in any one of claims 1 to 4, such that the collected liquid from the upper distillation zone circulates in the central zone of the catalytic zone, the major part of the liquid flowing into the lower distillation zone passes into the peripheral zone of the catalytic zone, and the vapour from the lower distillation zone circulating in the catalytic zone circulates in the peripheral zone of the catalytic zone.

6. A method as claimed in claim 5, such that the vapour from the lower distillation zone that circulates in the peripheral zone of the catalytic zone has no contact with the liquid circulating in the discharge means.

7. A method as claimed in any one of claims 1 to 6, such that the radial circulation means comprises at least one distribution arm.

8. A reactive distillation device comprising:
a) at least one catalytic reaction zone including at least one catalytic bed comprising the catalyst,
b) at least one distillation zone located below the aforementioned catalytic zone, or lower distillation zone,
c) at least another distillation zone located above the aforementioned catalytic zone, or upper distillation zone,
• at least one means intended for circulation of the major part of the liquid from bottom to top through the catalyst in each catalytic bed,
• at least one means intended for circulation of the vapour from bottom to top in the catalytic zone in such a way that said vapour is not in contact with the catalyst,
• at least one liquid discharge means comprising at least one line, so that the liquid flows into the lower distillation zone after passing through the catalytic bed,
• at least one collecting means,
said device being **characterized in that**:
• said at least one collecting means is adapted in such a way that the liquid from the upper distillation zone reaches the central zone of the catalytic zone, below each catalytic bed of the catalytic zone, said collecting means including, partly above the catalytic zone, a collecting zone, and said means including at least one line allowing the liquid to flow through the catalytic zone from top to bottom without being in contact with the catalyst,
and **in that** it comprises:
• at least one means intended for substantially radial circulation of the liquid below each bed of the catalytic zone so as to feed it into at least one liquid distribution zone.

9. A device as claimed in claim 8, such that the collecting zone of the collecting means allows release of the vapour.

10. A device as claimed in any one of claims 8 or 9, comprising at least one gaseous reagent feeding means for each catalytic bed of the catalytic zone.

11. A device as claimed in any one of claims 8 to 10, such that the discharge means intended for the liquid that flows into the lower distillation zone is located in the peripheral zone of the catalytic zone.

12. A device as claimed in any one of claims 8 to 11, such that the collecting means is located in the central zone of the catalytic zone, the discharge means is located in the peripheral zone of the catalytic zone and the circulation means intended for the vapour from the lower distillation zone is located in the central zone of the catalytic zone.

13. A device as claimed in any one of claims 8 to 12, such that the collecting means is located in the central zone of the catalytic zone, the discharge means is located in the peripheral zone of the catalytic zone and the circulation means intended for the vapour from the lower distillation zone is located in the peripheral zone of the catalytic zone.

14. A device as claimed in claim 13, such that the circulation means intended for the vapour from the lower distillation zone comprises at least one line, any line of said circulation means being distinct from any line of the liquid discharge means.

15. A device as claimed in any one of claims 8 to 14, such that the means intended for circulation of the liquid from bottom to top through the catalyst comprises at least one means for injecting said liquid into each catalytic bed of the catalytic zone.

16. A device as claimed in claim 15, such that any catalytic bed of the catalytic zone comprises at least one hydrogen feeding device.

17. A device as claimed in claim 16, such that the hydrogen feeding device is arranged before the liquid injection device.

18. A device as claimed in claim 16, such that the hydrogen feeding device is arranged level with the liquid injection device.

19. A device as claimed in claim 16, such that the hydrogen feeding device is arranged after the liquid injection device.

20. A device as claimed in any one of claims 8 to 19, such that the substantially radial circulation means comprises at least one distribution arm.

## Patentansprüche

1. Reaktives Destillationsverfahren, bei dem:
- Reagenzien alleine oder verdünnt eingeleitet werden, wobei eines der Reagenzien auf mindestens einem Niveau einer reaktiven Destillationszone überschüssig sein kann, die umfasst:
a) mindestens eine katalytische Reaktionszone, die mindestens ein Katalysebett umfasst, das den Katalysator enthält,
b) mindestens eine Destillationszone, die sich unter der vorhergehenden katalytischen Zone befindet, oder untere Destillationszone,
c) mindestens eine weitere Destillationszone, die sich über der vorhergehenden Destillationszone befindet, oder obere Destillationszone,
- derartige Destillationsbedingungen aufrecht erhalten werden, dass eine flüssige Phase und eine Dampfphase in der reaktiven Destillationszone vorhanden ist,
- der größte Teil der Flüssigkeit von unten nach oben durch den Katalysator in die katalytische Zone zum Zirkulieren gebracht wird,
- der größte Teil des Dampfes von unten nach oben in die katalytische Zone zum Zirkulieren gebracht wird, so dass der Dampf nicht mit dem Katalysator in Kontakt ist,
- ein Teil des gewünschten Reaktionsproduktes gesammelt wird,
- ein Teil eines möglichen Verdünnungsmittels und der Reagenzien gesammelt wird,
- der größte Teil der Flüssigkeit nach ihrem Durchströmen durch das Katalysebett durch mindestens ein Mittel zur Entleerung der Flüssigkeit wiedergewonnen wird, damit sich die Flüssigkeit in die untere Destillationszone entleert,
- und der größte Teil der Flüssigkeit aus der oberen Destillationszone gesammelt wird,
wobei das Verfahren **dadurch gekennzeichnet ist, dass**:
- der größte Teil der Flüssigkeit aus der oberen Destillationszone durch mindestens ein Sammelmittel gesammelt wird, so dass die Flüssigkeit in der zentralen Zone der katalytischen Zone unter jedem Katalysebett der katalytischen Zone ankommt, ohne mit dem Katalysator Kontakt zu haben,
- der größte Teil der Flüssigkeit im Wesentlichen radial unter dem Katalysebett durch mindestens ein im Wesentlichen radiales Zirkulationsmittel zum Zirkulieren gebracht wird, um ihn in mindestens eine Zone zur Verteilung der Flüssigkeit einzuleiten.

2. Verfahren nach Anspruch 1, umfassend mindestens die Einleitung eines gasförmigen Reagens für jedes Katalysebett der katalytischen Zone.

3. Verfahren nach einem der Ansprüche 1 und 2, bei dem die Flüssigkeit, die sich in die untere Destillationszone durch mindestens ein Entleerungsmittel entleert, zum größten Teil in der Umfangszone der katalytischen Zone umläuft.

4. Verfahren nach einem der Ansprüche 1 bis 3, bei dem Flüssigkeit aus der oberen Destillationszone, die gesammelt wird, in der zentralen Zone der katalytischen Zone zirkuliert, die Flüssigkeit, die sich in die untere Destillationszone entleert, zum größten Teil in der Umfangszone der katalytischen Zone umläuft, und der Dampf, der von der unteren Destillationszone kommt und in der katalytischen Zone zirkuliert, in der zentralen Zone der katalytischen Zone zirkuliert.

5. Verfahren nach einem der Ansprüche 1 bis 4, bei dem die Flüssigkeit aus der oberen Destillationszone die gesammelt wird, in der zentralen Zone der katalytischen Zone zirkuliert, die Flüssigkeit, die sich in die untere Destillationszone entleert, zum größten Teil in der Umfangszone der katalytischen Zone umläuft, und der Dampf, der von der unteren Destillationszone kommt und in der katalytischen Zone zirkuliert, in der Umfangszone der katalytischen Zone zirkuliert.

6. Verfahren nach Anspruch 5, bei dem der Dampf aus der unteren Destillationszone, der in der Umfangszone der katalytischen Zone zirkuliert, keinen Kontakt mit der in dem Entleerungsmittel zirkulierenden Flüssigkeit hat.

7. Verfahren nach einem der Ansprüche 1 bis 6, bei dem das radiale Zirkulationsmittel mindestens einen Verteilungsarm umfasst.

8. Reaktive Destillationsvorrichtung, umfassend:
a) mindestens eine katalytische Reaktionszone, die mindestens ein Katalysebett umfasst, das den Katalysator enthält,
b) mindestens eine Destillationszone, die sich unter der vorhergeschalteten katalytischen Zone befindet, oder untere Destillationszone,
c) mindestens eine weitere Destillationszone,die sich über der vorhergeschalteten Destillationszone befindet, oder obere Destillationszone,
- mindestens ein Mittel, um den größten Teil der Flüssigkeit von unten nach oben durch den Katalysator in jedem Katalysebett zum Zirkulieren zu bringen,
- mindestens ein Mittel, um den Dampf von unten nach oben in die katalytische Zone zum Zirkulieren zu bringen, so dass der Dampf nicht mit dem Katalysator in Kontakt ist,
- mindestens ein Mittel zur Entleerung der Flüssigkeit, das mindestens eine Leitung umfasst, damit sich die Flüssigkeit nach ihrem Durchströmen durch das Katalysebett in die untere Destillationszone entleert,
wobei die Vorrichtung **dadurch gekennzeichnet ist, dass**:
- das mindestens eine Sammelmittel derart ausgeführt ist, dass die Flüssigkeit aus der oberen Destillationszone in der zentralen Zone der katalytischen Zone unter jedem Katalysebett der katalytischen Zone ankommt, wobei das Sammelmittel in dem Teil über der katalytischen Zone eine Sammelzone umfasst, und wobei das Mittel mindestens eine Leitung umfasst, die das Durchströmen der Flüssigkeit in der katalytischen Zone von oben nach unten ermöglicht, ohne mit dem Katalysator Kontakt zu haben,
und dass es umfasst:
- mindestens ein im Wesentlichen radiales Mittel zur Zirkulation der Flüssigkeit unter jedem Bett der katalytischen Zone, um sie in mindestens eine Zone zur Verteilung der Flüssigkeit einzuleiten.

9. Vorrichtung nach Anspruch 8, bei der die Sammelzone des Sammelmittels die Freigabe des Dampfes ermöglicht.

10. Vorrichtung nach einem der Ansprüche 8 oder 9, umfassend mindestens ein Mittel zur Einleitung eines gasförmigen Reagens für jedes Katalysebett der katalytischen Zone.

11. Vorrichtung nach einem der Ansprüche 8 bis 10, bei dem das Mittel zur Entleerung der Flüssigkeit, die sich in die untere Destillationszone entleert, in der Umfangszone der katalytischen Zone angeordnet ist.

12. Vorrichtung nach einem der Ansprüche 8 bis 11, bei dem das Sammelmittel in der zentralen Zone der katalytischen Zone, das Entleerungsmittel in der Umfangszone der katalytischen Zone und das Zirkulationsmittel des Dampfes aus der unteren Destillationszone in der zentralen Zone der katalytischen Zone angeordnet ist.

13. Vorrichtung nach einem der Ansprüche 8 bis 12, bei dem das Sammelmittel in der zentralen Zone der katalytischen Zone, das Entleerungsmittel in der Umfangszone der katalytischen Zone und das Zirkulationsmittel des Dampfes aus der unteren Destillationszone in der Umfangszone der katalytischen Zone angeordnet ist.

14. Vorrichtung nach Anspruch 13, bei der das Zirkulationsmittel des Dampfes aus der unteren Destillationszone mindestens eine Leitung umfasst, wobei jede Leitung des Zirkulationsmittels von jeder Leitung des Mittels zur Entleerung der Flüssigkeit getrennt ist.

15. Vorrichtung nach einem der Ansprüche 8 bis 14, bei der das Mittel zur Zirkulation der Flüssigkeit von unten nach oben durch den Katalysator mindestens ein Mittel zum Einspritzen der Flüssigkeit in jedes Katalysebett der katalytischen Zone umfasst.

16. Vorrichtung nach Anspruch 15, bei der jedes Katalysebett der katalytischen Zone mindestens eine Vorrichtung zur Einleitung von Wasserstoff umfasst.

17. Vorrichtung nach Anspruch 16, bei der die Vorrichtung zur Einleitung von Wasserstoff vor der Vorrichtung zum Einspritzen von Flüssigkeit angeordnet ist.

18. Vorrichtung nach Anspruch 16, bei der die Vorrichtung zur Einleitung von Wasserstoff im Bereich der Vorrichtung zum Einspritzen von Flüssigkeit angeordnet ist.

19. Vorrichtung nach Anspruch 16, bei der die Vorrichtung zur Einleitung von Wasserstoff nach der Vorrichtung zum Einspritzen von Flüssigkeit angeordnet ist.

20. Vorrichtung nach einem der Ansprüche 8 bis 19, bei der das im Wesentlichen radiale Zirkulationsmittel mindestens einen Verteilungsarm umfasst.
